(19) Europäisches Patentamt / European Patent Office / Office européen des brevets

(11) **EP 4 427 613 A1**

(12) **EUROPEAN PATENT APPLICATION**
published in accordance with Art. 153(4) EPC

(43) Date of publication:
**11.09.2024 Bulletin 2024/37**

(21) Application number: **21963186.8**

(22) Date of filing: **02.11.2021**

(51) International Patent Classification (IPC):
**A24F 40/53** (2020.01)

(52) Cooperative Patent Classification (CPC):
**A24F 40/53**

(86) International application number:
**PCT/JP2021/040355**

(87) International publication number:
**WO 2023/079588 (11.05.2023 Gazette 2023/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Japan Tobacco, Inc.**
**Tokyo, 105-6927 (JP)**

(72) Inventors:
• **JOMURA, Naohiro**
  **Tokyo 130-8603 (JP)**
• **NAGATA, Hisanori**
  **Tokyo 130-8603 (JP)**

(74) Representative: **Hoffmann Eitle**
**Patent- und Rechtsanwälte PartmbB**
**Arabellastraße 30**
**81925 München (DE)**

(54) **FLAVOR INHALER OR AEROSOL GENERATING DEVICE, AND CONTROL METHOD THEREFOR AND PROGRAM THEREFOR**

(57) Provided is a flavor inhaler that delivers a psychological effect that is effective for a user. A device (200), which is a flavor inhaler or an aerosol generating device, comprises a sense stimulating element (210), a heart rate acquisition unit (220) for acquiring the heart rate of a user, and a control unit (240) for operating the sense stimulation element. The operation of the sense stimulation element (210) has periodicity. The control unit (240) is configured to operate the sense stimulation element (210) such that the operation period of the sense stimulation element (210) is based on the heart rate obtained by the heart rate acquisition unit (220).

Fig. 2

## Description

### TECHNICAL FIELD

The present application relates to a flavor inhaler or an aerosol generating device (hereinafter referred to as a "flavor inhaler or the like"). More specifically, the present application relates to a flavor inhaler or the like including a sensory stimulation device that operates on the basis of a user's heart rate.

Flavor inhalers are devices for inhaling flavors and include heated flavor inhalers (those that produce flavors through heating) and non-heated flavor inhalers (e.g., those that produce flavors through ultrasonic atomization). More specifically, flavor inhalers include, but are not limited to, for example, electronic cigarettes, heated cigarettes, and conventional cigarettes. "Aerosol generating devices" refer to apparatuses for inhaling generated aerosols and include heated aerosol generating devices (those that produce aerosols through heating) and non-heated aerosol generating devices (e.g., those that produce aerosols through ultrasonic atomization). More specifically, aerosol generating devices include, but are not limited to, for example, electronic cigarettes, heated cigarettes, and medical nebulizers. Flavor inhalers or the like also include so-called RRPs (reduced-risk products).

### BACKGROUND ART

The act of smoking cigarettes (conventional cigarettes) or the like is conventionally known to produce psychological effects such as arousal and calming in users. In recent years, flavor inhalers or the like equipped with electronic devices, such as heated cigarettes and electronic cigarettes, have become popular as alternatives to cigarettes. Attempts have been made to use these flavor inhalers or the like to measure a user's heart rate and provide a certain function for the user (e.g., refer to PTL 1).

For example, provision of various values for a user by measuring the user's heart rate is being examined. One such example is, as in PTL 1, to notify a user or a medical institution when the user's heart rate exceeds a certain value. There are also users, on the other hand, who seek psychological effects from smoking as one of various values. For such users, there is a need for flavor inhaler that enhance psychological effects.

### CITATION LIST

### PATENT LITERATURE

PTL 1: Japanese Unexamined Patent Application Publication (Translation of PCT Application) No. 2021-500002

## SUMMARY OF INVENTION

### TECHNICAL PROBLEM

The present invention has been conceived in view of the above circumstances and aims to provide a flavor inhaler or the like that produces effective psychological effects for a user.

### SOLUTION TO PROBLEM

In order to solve the above problem, an embodiment of the present invention provides a device that is a flavor inhaler or an aerosol generating device. The device includes a sensory stimulation device, a heart rate obtaining unit for obtaining a user's heart rate, and a controller for operating the sensory stimulation device. The sensory stimulation device operates cyclically. The controller is configured to operate the sensory stimulation device such that a cycle of the operation of the sensory stimulation device is based on the heart rate obtained by the heart rate obtaining unit.

In the embodiment, the heart rate obtaining unit may be a heart rate sensor, and the heart rate sensor may be a pulse wave sensor.

In the embodiment, the heart rate obtaining unit may be a communicator configured to obtain the heart rate from an external device.

In the embodiment, the cycle of the operation of the sensory stimulation device may be within a range of 80% to 120% of a cycle corresponding to the obtained heart rate.

The device as the embodiment may have an arousal mode and a calming mode. The controller may also be configured to operate the sensory stimulation device such that the cycle of the operation of the sensory stimulation device becomes, in the arousal mode, shorter than a cycle corresponding to the obtained heart rate and, in the arousal mode, longer than the cycle corresponding to the obtained heart rate.

In the embodiment, operation intensity of the sensory stimulation device in the arousal mode may be greater than operation intensity of the sensory stimulation device in the calming mode.

The device as the embodiment may determine whether the user has been aroused and/or calmed by comparing the heart rate at a beginning of the obtaining and the heart rate at an end of the obtaining and notify of a result of the determination.

In the embodiment, a period of time for which the sensory stimulation device operates may be longer than or equal to 0.05 second and shorter than or equal to 0.5 second in one cycle of the operation of the sensory stimulation device.

In the embodiment, the sensory stimulation device may be a vibrator, a light-emitting device, and/or an acoustic device.

The heart rate obtained by the heart rate obtaining unit may be a moving average of the user's heart rate measured in a certain period of time.

In order to solve the above problem, another embodiment of the present invention provides a method for operating a device that is a flavor inhaler or an aerosol generating device. The device comprises a sensory stimulation device, a heart rate obtaining unit for obtaining a user's heart rate, and a controller for operating the sensory stimulation device. The sensory stimulation device operates cyclically. The method comprises a step of operating, by the controller, the sensory stimulation device such that a cycle of the operation of the sensory stimulation device is based on the heart rate obtained by the heart rate obtaining unit.

In order to solve the above problem, yet another embodiment of the present invention provides a program causing a processor of a flavor inhaler or an aerosol generating device to perform the method.

ADVANTAGEOUS EFFECTS OF INVENTION

According to the embodiments of the present invention, effective psychological effects can be produced for a user of a flavor inhaler or the like through sensory stimulation.

BRIEF DESCRIPTION OF DRAWINGS

[Fig. 1A] Fig. 1A is a schematic diagram schematically illustrating a configuration example of a flavor inhaler or the like according to an embodiment of the present invention.
[Fig. 1B] Fig. 1B is a schematic diagram schematically illustrating another configuration example of the flavor inhaler or the like according to the embodiment of the present invention.
[Fig. 2] Fig. 2 is a schematic diagram schematically illustrating another configuration example of the flavor inhaler or the like according to the embodiment of the present invention.
[Fig. 3A] Fig. 3A is a diagram illustrating a position at which a heart rate sensor included in the flavor inhaler or the like is provided.
[Fig. 3B] Fig. 3B is a diagram illustrating another position at which the heart rate sensor included in the flavor inhaler or the like is provided.
[Fig. 4] Fig. 4 is a flowchart illustrating an exemplary process performed by a controller included in the flavor inhaler or the like.
[Fig. 5A] Fig. 5A is a flowchart illustrating another exemplary process performed by the controller included in the flavor inhaler or the like.
[Fig. 5B] Fig. 5B is a flowchart illustrating another exemplary process performed by the controller included in the flavor inhaler or the like.

DESCRIPTION OF EMBODIMENTS

1 Embodiment of Prevent Invention

An embodiment of the present invention is a flavor inhaler or the like having a function of giving sensory stimulation to a user.

1-1 First Configuration Example

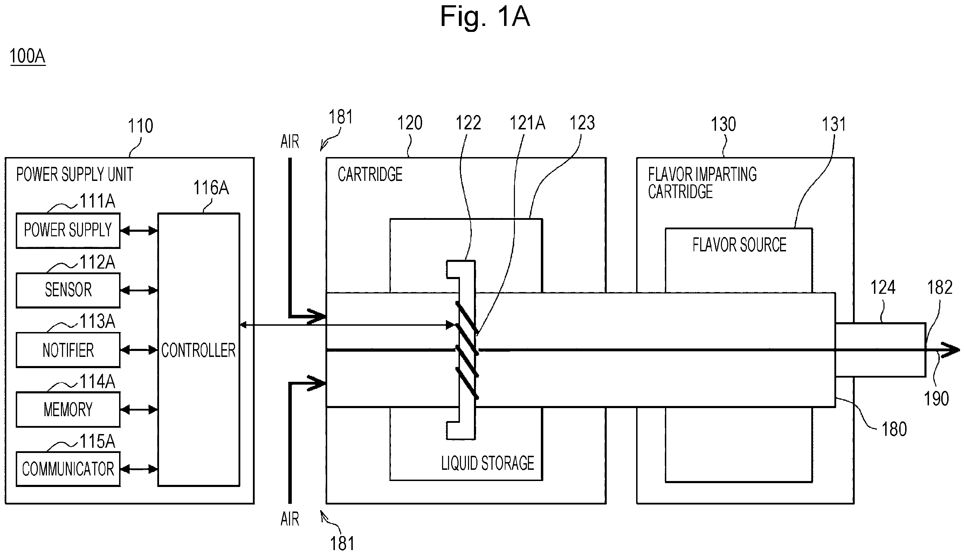

Fig. 1A is a schematic diagram schematically illustrating a first configuration example of the flavor inhaler or the like. As illustrated in Fig. 1A, a flavor inhaler or the like 100A according to this configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. An airflow path 180 is formed in the cartridge 120 and the flavor imparting cartridge 130.

The power supply 111A stores electric power. The power supply 111A supplies electric power to the structural elements of the flavor inhaler or the like 100A under the control of the controller 116A. The power supply 111A may be a rechargeable battery such as a lithium-ion secondary battery.

The sensor 112A obtains various pieces of information regarding the flavor inhaler or the like 100A. The sensor 112A may be a pressure sensor such as a microphone condenser, a flowrate sensor, or a temperature sensor, and obtain a value based on the user's inhalation. The sensor 112A may also include an input device that receives information input by the user, such as a button or a switch. The sensor 112A may also include a heart rate sensor for obtaining a heart rate of the user of the flavor inhaler or the like 100A. A plurality of heart rate sensors may be provided, instead.

The notifier 113A notifies the user of information. The notifier 113A according to the present embodiment includes a sensory stimulation device configured to give sensory stimulation to the user. The sensory stimulation device may be a light-emitting device that emits light, an acoustic device that outputs sound, and/or a vibrator that vibrates. That is, the sensory stimulation device may be an acoustic device, a light-emitting device, a vibrator, or a combination of two or more of these. The notifier 113A may include a display device that displays messages.

The memory 114A stores various pieces of information for operating the flavor inhaler or the like 100A. The memory 114A is achieved, for example, by a nonvolatile storage medium such as flash memory. The memory 114A may include a volatile memory that provides a work area for the control performed by the controller 116A. An example of the information stored in the memory 114A is the user's heart rate, which will be described later.

The communicator 115A may include a communication interface (includes a communication electronic circuit or the like that can include a communication module and an antenna; the same holds in the following description) that conforms to a predetermined LPWA wireless communication standard or a wireless communication standard having similar restrictions. Such a communication standard may be Sigfox, LoRA-WAN, or the like. The communicator 115A may be a communication interface capable of communication in conformity with any wired or wireless communication standard, instead. Such a communication standard may be, for example, Wi-Fi (registered trademark), Bluetooth (registered trademark), or the like. The communicator 115A may be configured to communicate with external devices (not illustrated).

The controller 116A functions as an arithmetic processing device and a control device, and controls overall operations of the flavor inhaler or the like 100A in accordance with various programs. The controller 116A is achieved, for example, by an electronic circuit such as a CPU (central processing unit) or a microprocessor. The controller 116A may include a converter 117A, which will be described in detail later.

The liquid storage 123 stores an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the flavor inhaler or the like 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, capillary action of the wick guides the aerosol source stored in the liquid storage 123.

The heater 121A heats the aerosol source to atomize the aerosol source and generate the aerosol. In the example illustrated in Fig. 1A, the heater 121A is a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. The supply of the electric power may then be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181, which is an inlet of air into the airflow path 180, and an air outlet hole 182, which is an outlet of the air from the airflow path 180, at two ends thereof, respectively. The liquid guide 122 is provided in the airflow path 180 on an upstream side (closer to the air inlet hole 181), and the flavor source 131 is provided in the airflow path 180 on a downstream side (closer to the air outlet hole 182). Air flowing in from the air inlet hole 181 as the user inhales mixes with the aerosol generated by the heater 121A. As indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is a member to be held in the user's mouth during inhalation. The mouthpiece 124 has the air outlet hole 182. When the user inhales with the mouthpiece 124 in his/her mouth, the mixture fluid of the aerosol and the air enters the oral cavity of the user.

A configuration example of the flavor inhaler or the like 100A has been described. The flavor inhaler or the like 100A is not limited to the above configuration, and may be configured in various ways as exemplified below.

In an example, the flavor inhaler or the like 100A need not include the flavor imparting cartridge 130. In this case, the cartridge 120 is provided with the mouthpiece 124.

In another example, the flavor inhaler or the like 100A may include aerosol sources of a plurality of types. An aerosol of yet another type may be generated by mixing aerosols of a plurality of types generated from the aerosol sources of the plurality of types in the airflow path 180 and causing a chemical reaction.

In addition, means for atomizing the aerosol source is not limited to the heating by the heater 121A. For example, the means for atomizing the aerosol source may be vibration atomization or induction heating, instead.

1-2 Second Configuration Example

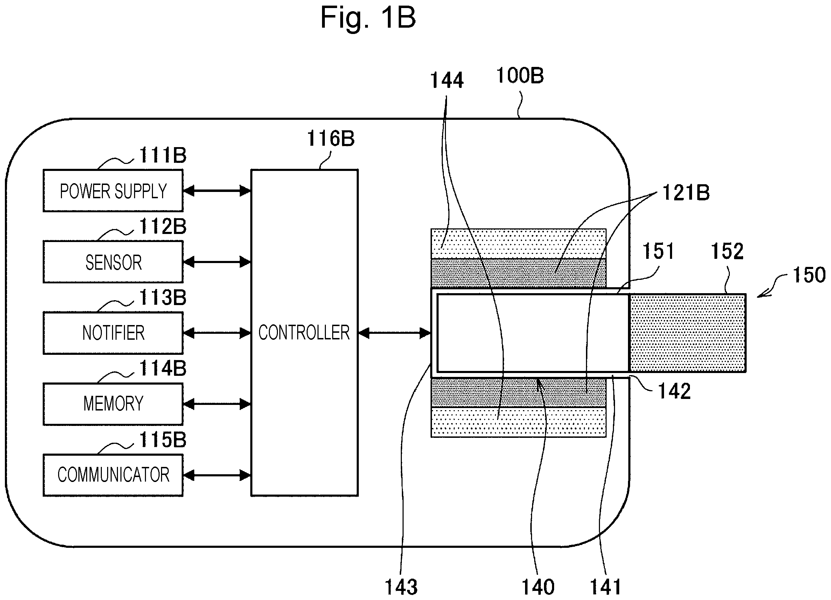

Fig. 1B is a schematic diagram of a second configuration example of the flavor inhaler or the like. As illustrated in Fig. 1B, a flavor inhaler or the like 100B according to this configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the corresponding structural elements included in the flavor inhaler or the like 100A according to the first configuration example.

The holder 140 has an internal space 141 and holds a stick substrate 150 while accommodating part of the stick substrate in the internal space 141. The stick substrate 150, too, is an example of a "refill". The holder 140 has an opening 142 that allows the internal space 141 to communicate with the outside and holds the stick substrate 150 inserted into the internal space 141 from the opening 142. For example, the holder 140 may be a tubular body having the opening 142 and a bottom 143 at two ends thereof and defines the pillar-shaped internal space 141. The holder 140 also has a function of defining a path of air supplied to the stick substrate 150. The bottom 143, for example, has an air inlet hole that serves as an inlet of air into the airflow path. The opening 142, on the other hand, has an air outlet hole that serves as an outlet of air from the airflow path.

The stick substrate 150 includes a substrate 151 and an inhalation port 152. The substrate 151 includes an aerosol source. In this configuration example, the aerosol source is not limited to a liquid, and may be a solid, instead. With the stick substrate 150 held by the holder 140, at least a part of the substrate 151 is accommodated in the internal space 141, and at least a part of the inhalation port 152 protrudes from the opening 142. When the user inhales with the inhalation port 152, which protrudes from the opening 142, in his/her mouth, air flows into the internal space 141 from the air inlet hole (not illustrated) and reaches inside the user's mouth along with an aerosol generated from the substrate 151.

The heater 121B has the same configuration as the heater 121A according to the first configuration example. In the example illustrated in Fig. 1B, however, the heater 121B has a film-like shape and surrounds an outer circumference of the holder 140. When the heater 121B produces heat, the substrate 151 of the stick substrate 150 is heated from the outer circumference, thereby generating the aerosol.

The heat insulator 144 prevents heat from transferring from the heater 121B to the other structural elements. For example, the heat insulator 144 may be a vacuum heat insulator, an aerogel heat insulator, or the like.

The configuration example of the flavor inhaler or the like 100B has been described. The flavor inhaler or the like 100B is not limited to the above configuration, and may be configured in various ways as exemplified below.

In an example, the heater 121B may have a blade-like shape, and may be disposed in such a way as to protrude from the bottom 143 of the holder 140 into the internal space 141. In this case, the heater 121B having the blade-like shape is inserted into the substrate 151 of the stick substrate 150 and heats the substrate 151 of the stick substrate 150 from the inside. In another example, the heater 121B may be disposed in such a way as to cover the bottom 143 of the holder 140. The heater 121B may be implemented as a combination of two or more selected from a first heater that covers the outer circumference of the holder 140, a second heater having a blade-like shape, and a third heater that covers the bottom 143 of the holder 140.

In another example, the holder 140 may include an opening/closing mechanism, such as a hinge, that opens and closes a part of an outer shell that defines the internal space 141. In addition, the holder 140 may sandwich the stick substrate 150 inserted into the internal space 141 by opening and closing the outer shell. In this case, the heater 121B may be disposed at a sandwiching position of the holder 140 and produce heat while pressing the stick substrate 150.

In addition, means for atomizing the aerosol source is not limited to the heating by the heater 121B. For example, the means for atomizing the aerosol source may be induction heating, instead.

The flavor inhaler or the like 100B may also include the heater 121A, the liquid guide 122, the liquid storage 123, and the airflow path 180 according to the first configuration example, and the air outlet hole 182 of the airflow path 180 may also serve as an air inlet hole leading to the internal space 141. In this case, a mixture fluid of the air and an aerosol generated by the heater 121A flows into the internal space 141, mixes further with an aerosol generated by the heater 121B, and then reaches the user's oral cavity.

1-3 Simplified Configuration Example

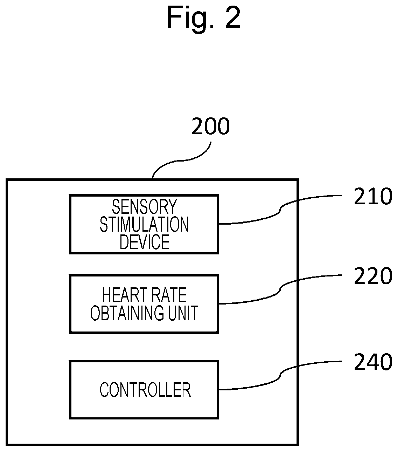

Fig. 2 is a schematic diagram schematically illustrating an example of a simplified configuration where only structural elements especially relating to the embodiment of the present invention are extracted from the above-described flavor inhaler or the like 100A or 100B. 200, therefore, denotes the flavor inhaler or the like 100A or 100B.

210 denotes the above-described sensory stimulation device included in the notifier 113A or 113B. The sensory stimulation device 210 may be considered as a structural element separate from the notifier 113A or 113B.

220 denotes a heart rate obtaining unit for obtaining a heart rate of a user of the flavor inhaler or the like 200. The heart rate obtaining unit 220 may be the above-described heart rate sensor, which is included in the sensor 112A or 112B, for obtaining the heart rate of the user of the flavor inhaler or the like 200. Alternatively, the heart rate obtaining unit 220 may be the communicator 115A or 115B configured to obtain the heart rate of the user of the flavor inhaler or the like 200 from an external device (not illustrated). The external device may include a heart rate sensor or be configured to communicate with another external device (not illustrated) including a heart rate sensor. More specifically, the external device may be a smartphone, a wearable device (a smartwatch etc.), a heart rate monitor having a communication function, or the like, but is not limited to these.

240 denotes the controller 116A or 116B.

1-4 Sensory Stimulation Device 210

As described above, the sensory stimulation device 210 may be a light-emitting device that emits light, an acoustic device that outputs sound, and/or a vibrator that vibrates, but it is known that a vibrator is preferable as a sensory stimulation device for the purpose of arousing or calming the user in the present embodiment, which will be described later. The vibrator may specifically be a vibration motor, a linear vibration actuator, or the like. As described later, the sensory stimulation device 210 is controlled such that the sensory stimulation device 210 operates cyclically.

1-5 Heart Rate Obtaining Unit 220

1-5-1 Types of Heart Rate Sensor

The heart rate sensor that is the heart rate obtaining unit 220 and the heart rate sensor included in the external device or another external device that communicates with the external device (hereinafter referred to as an "external device or the like") may be any known sensors capable of detecting a heart rate through electrocardiography, photoplethysmography, sphygmomanometry, phonocardiography, or the like. When the heart rate obtaining unit 220 is a heart rate sensor and therefore the flavor inhaler or the like 200 includes the heart rate sensor, the heart rate sensor is preferably a pulse wave sensor, especially a reflective pulse wave sensor that employs photoplethysmography. The heart rate sensor included in the flavor inhaler or the like 200 as the heart rate obtaining unit 220 will be referred to as a "heart rate sensor 220" hereinafter.

1-5-2 Position of Heart Rate Sensor

The heart rate sensor 220 may be provided at an appropriate position that suits a type thereof and a shape of the flavor inhaler or the like 200. When the heart rate sensor 220 is a pulse wave sensor, especially a reflective pulse wave sensor that employs photoplethysmography, for example, the sensor is preferably provided at a position on the flavor inhaler or the like 200 where the user can touch the sensor with one of his/her fingers. This is because it is generally difficult for a pulse wave sensor to detect a pulse wave, that is, a heart rate, from a palm.

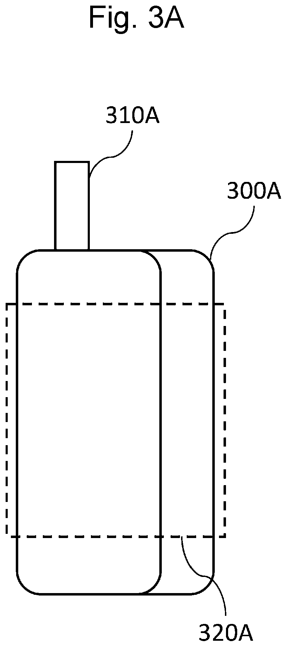

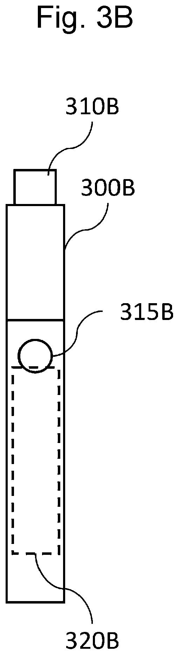

Figs. 3A and 3B are diagrams illustrating examples of a position at which a pulse wave sensor used as the heart rate sensor 220 is provided and that suits the shape of the flavor inhaler or the like 200.

300A and 300B denote the flavor inhaler or the like 200.

310A denotes the stick substrate 150, and 310B denotes the mouthpiece 124.

315B denotes a button.

320A and 320B denote appropriate ranges within which the heart rate sensor 220 can be provided and that suit shapes of the flavor inhalers or the like 300A and 300B, respectively. That is, in the case of the flavor inhalers or the like 300A and 300B, the heart rate sensor 220 is preferably provided at any position within the ranges 320A and 320B, respectively. The range 320A includes a side surface (a part in a direction perpendicular to a page) and a back surface (a part not illustrated in the page) of the flavor inhaler or the like 300A. The range 320B is intended as a back surface (a part not illustrated in the page) of the flavor inhaler or the like 300B where the button 315B does not exist.

A mark may be given to the position on the flavor inhaler or the like 200 at which the heart rate sensor 220 is provided to inform the user of the position of the heart rate sensor 220.

A plurality of heart rate sensors 220 may be provided for the flavor inhaler or the like 200. The heart rate, which will be described later, measured by the heart rate sensor 220, therefore, may be a heart rate measured by one of the plurality of heart rate sensors. Alternatively, the heart rate, which will be described later, measured by the heart rate sensor may be an average of heart rates measured by the plurality of heart rate sensors.

The heart rate sensor included in the external device or the like may be provided at an appropriate position on the external device or the like in accordance with a type thereof and a shape of the external device or the like.

1-5-3 Method for Obtaining Heart Rate

In the present embodiment, a heart rate is defined as the number of heartbeats in a certain period of time (usually 1 minute = 60 seconds). The user's heart rate may be obtained by any method, but an exemplary method will be described hereinafter.

The user's heart rate can be measured with the following expression using the number of heartbeats of the user detected in the certain period of time.

$$A = a \times (60/T) \qquad (1)$$

Here, A denotes the heart rate to be measured, a denotes the number of heartbeats of the user detected in the certain period of time, and T denotes the length of the certain period of time (unit is seconds).

The user's heart rate obtained by the heart rate obtaining unit 220 may be a heart rate measured as above.

Alternatively, the user's heart rate obtained by the heart rate obtaining unit 220 may be a moving average of heart rates measured as above a plurality of times. For example, a heart rate obtained after n-th measurement of the heart rate may be an average of a heart rate in the n-th measurement, a heart rate in (n - 1)th measurement, ..., and a heart rate in (n - 1)th measurement. Here, 0 < i < n. In other words, the user's heart rate obtained by the heart rate obtaining unit 220 may be an average of heart rates measured past (i + 1) times of measurement. A value of i may be different each time a heart rate is obtained. For example, a heart rate obtained in first measurement may be an average (i = 2) of heart rates measured in past three times of measurement, a heart rate obtained in second measurement may be an average (i = 3) of heart rates measured in past four times of measurement, ..., and a heart rate obtained in j-th measurement may be an average (i = j + 1) of heart rates measured in past (j + 2) times of measurement. In this case, an upper limit may be provided for the number of times of past measurement used to obtain an average. For example, the upper limit may be 10, and heart rates obtained in eighth and later measurement may be averages of heart rates measured in past 10 times of measurement.

When a heart rate is measured as above a plurality of times, the length (T) of the certain period of time in each measurement may be the same or different.

1-6 Controller 240

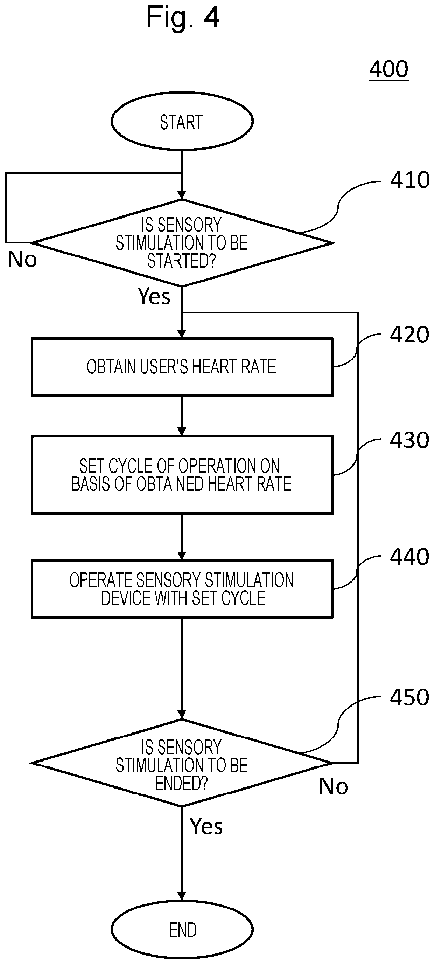

Fig. 4 is a flowchart illustrating an exemplary process 400 performed by the controller 240. In the exemplary process 400, the sensory stimulation device 210 operates and is controlled such that the sensory stimulation device 210 operates cyclically.

410 denotes a step of determining whether to start sensory stimulation for the user.

The sensory stimulation for the user can be linked to the user's inhalation with the flavor inhaler or the like 200. In step 410, the flavor inhaler or the like 200 determines whether the user has started inhalation and, if determining that the user has started inhalation, can determine that the sensory stimulation is to start. Any method for determining whether the user has started inhalation may be used, but, for example, the above-described pressure sensor that is included in the flavor inhaler or the like 200 and that is configured to detect changes in pressure caused by inhalation may be used for the determination. More specifically, if pressure measured by the pressure sensor falls below a certain threshold, for example, the flavor inhaler or the like 200 can determine that inhalation has started.

Alternatively, in step 410, when the heating by the above-described heater 121A or 121B included in the flavor inhaler or the like 200 has started, the flavor inhaler or the like 200 can determine that the sensory stimulation is to start. Alternatively, when a certain operation is performed on the above-described input device, such as a button or a switch, included in the flavor inhaler or the like 200, the flavor inhaler or the like 200 can determine that the sensory stimulation is to start.

If the flavor inhaler or the like 200 determines that the sensory stimulation is to start, the process proceeds to step 420, and if not, the process returns to step 410.

420 denotes a step of obtaining the user's heart rate using the heart rate obtaining unit 220. A method for obtaining the user's heart rate is as described above. Step 420 may include a step of obtaining the user's heart rate using the heart rate sensor 220 (the heart rate sensor included in the flavor inhaler or the like 200 as the heart rate obtaining unit 220). Alternatively, step 420 may include a step of obtaining the user's heart rate from an external device. Measurement of the user's heart rate for obtaining the user's heart rate (described above) may be performed in step 420, but may be repeatedly performed asynchronously in parallel with the exemplary process 400. The measurement of the user's heart rate, for example, may start when the flavor inhaler or the like 200 is turned on and end when the flavor inhaler or the like 200 is turned off. Alternatively, the measurement of the user's heart rate may start when the heating by the above-described heater 121A or 121B included in the flavor inhaler or the like 200 starts and end when the heating ends. Alternatively, the measurement of the user's heart rate may start when the certain operation is performed on the above-described input device, such as a button or a switch, included in the flavor inhaler or the like 200 and end when another certain operation is performed. The obtained heart rate can be displayed on the above-described display device that displays messages.

430 denotes a step of setting a cycle of the operation of the sensory stimulation device 210 on the basis of the user's heart rate obtained.

The cycle of the operation of the sensory stimulation device 210 is preferably within a range of 80% to 120% of a cycle corresponding to the obtained heart rate and more preferably within a range of 90% to 110% of the cycle. When the "heart rate" is defined as the number of heartbeats in a certain period of time as described above, the "cycle corresponding to the heart rate" may be obtained using the following expression.

$$P = T_0/A \quad (2)$$

Here, P denotes the cycle (unit is seconds) corresponding to the heart rate, $T_0$ denotes the length of the certain period of time (unit is seconds; usually 60 seconds), and A denotes the heart rate.

The flavor inhaler or the like 200 may use an arousal mode and a calming mode, and the user can set a mode by any method, that is, for example, by performing the certain operation on the above-described input device, such as a button or a switch, included in the flavor inhaler or the like 200. When the flavor inhaler or the like 200 is in the arousal mode, a set cycle of the operation of the sensory stimulation device 210 is preferably shorter than a cycle corresponding to an obtained heart rate. More specifically, the set cycle is preferably 80% to 99% of the cycle corresponding to the obtained heart rate and more preferably 90% to 95% of the cycle. When the flavor inhaler or the like 200 is in the calming mode, a set cycle of the operation of the sensory stimulation device 210 is preferably longer than a cycle corresponding to an obtained heart rate. More specifically, the set cycle is preferably 101% to 120% of the cycle corresponding to the obtained heart rate and more preferably 105% to 110% of the cycle.

440 denotes a step of operating the sensory stimulation device 210 with the set cycle.

In one cycle of the operation of the sensory stimulation device 210, a period of time for which the sensory stimulation device 210 actually operates is preferably close to time taken for the user's heart to beat once (time for which the heart actually moves, not time between heartbeats). More specifically, the time for which the sensory stimulation device 210 actually operates in one cycle of the operation of the sensory stimulation device 210, therefore, may be preferably 0.05 to 0.5 second and more preferably 0.2 to 0.3 second.

When the flavor inhaler or the like 200 is in the arousal mode, operation intensity of the sensory stimulation device 210 may be higher than in the calming mode.

The sensory stimulation device 210 can be used to make some notifications for the user (a notification about a start of heating of the aerosol source, a notification about preliminary heating of the aerosol source, a notification about an end of heating of the aerosol source, etc.), but is preferably configured to be able to distinguish an operation relating to such notification from the operation in step 440. When a vibrator is used as the sensory stimulation device 210, for example, modes (vibration frequency, vibration intensity, a vibration pattern, etc.) for vibration relating to the former operation and vibration relating to the latter operation are preferably different from each other.

450 denotes a step of determining whether to end the sensory simulation for the user.

As described above, the sensory stimulation for the user may be linked to the user's inhalation with the flavor inhaler or the like 200. In step 450, therefore, the flavor inhaler or the like 200 determines whether the user's inhalation has ended and if so, can determine that the sensory stimulation is to end. Any method for determining whether the user's inhalation has ended may be used, but, for example, the above-described pressure sensor that is included in the flavor inhaler or the like 200 and that is configured to detect changes in pressure caused by inhalation may be used for the determination. More specifically, when pressure measured by the pressure sensor exceeds a certain threshold, for example, the flavor inhaler or the like 200 can determine that the user's inhalation has ended.

Alternatively, in step 450, when the heating by the above-described heating 121A or 121B included in the flavor inhaler or the like 200 ends, the flavor inhaler or the like 200 can determine that the sensory stimulation is to end. Alternatively, when the certain operation is performed on the above-described input device, such as a button or a switch, included in the flavor inhaler or the like 200, the flavor inhaler or the like 200 can determine that the sensory stimulation is to end.

If the flavor inhaler or the like 200 determines that the sensory stimulation is to end, the process ends, and if not, the process returns to step 420.

It should be understood from steps 420 to 440 that the controller 240 operates the sensory stimulation device 210 such that the cycle of the operation of the sensory stimulation device 210 is based on the heart rate obtained by the heart rate obtaining unit 220.

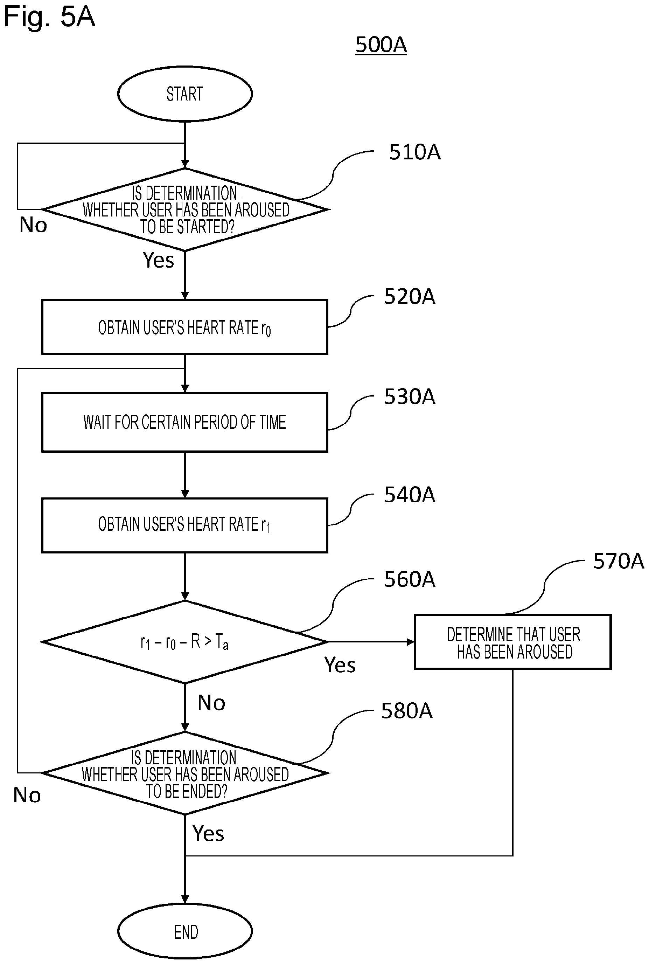

As a result of the sensory stimulation, the controller 240 can perform a process for determining whether the user has been aroused. Fig. 5A is a flowchart illustrating an exemplary process 500A performed by the controller 240 to determine whether the user has been aroused. The exemplary process 500A may be performed in parallel with the exemplary process 400.

510A denotes a step of determining whether to start a determination whether the user has been aroused. Any reference may be used for the determination whether to start the determination whether the user has been aroused. When the flavor inhaler or the like 200 enters the arousal mode, for example, it may be determined that the determination whether the user has been aroused is to start. Alternatively, it may be determined that the determination whether to start the determination whether the user has been aroused is to start, for example, when the flavor inhaler or the like 200 is in the arousal mode, when it has been determined that the user has started inhalation, when heating of the flavor inhaler or the like 200 has started, or when the certain operation has been performed on the input device such as a button or a switch. If it is determined that the determination whether the user has been aroused is to start, the process proceeds to step 520A, and if not, the process returns to step 510A.

520A denotes a step of obtaining the user's heart rate using the heart rate obtaining unit 220. The heart rate obtained in this step will be denoted by $r_0$ hereinafter. This step may be the same as step 420. Alternatively, the heart rate obtained in the previous step 420 may be used as the heart rate obtained in this step.

530A denotes a step of waiting for a certain period of time before performing processing. For example, the length of the certain period of time may be set on the basis of the length of time taken, typically or uniquely by the user, to take one puff so that step 520A is performed at a beginning of one puff and step 540A, which will be described later, is performed at an end of one puff.

540A denotes a step of obtaining the user's heart rate using the heart rate obtaining unit 220. The heart rate obtained in this step will be denoted by ri hereinafter. This step may be the same as step 520A.

550A denotes a step of comparing the heart rate $r_0$ obtained in step 520A and the heart rate ri obtained in step 540A and determining whether the heart rate has increased.

More specifically, comparison based on the following expression can be performed in this step.

$$r_1 - r_0 - R > T_a \qquad (3)$$

Here, R is a correction term larger than 0. That is, since the heart rate generally increases due to smoking, the correction term is provided to offset the increase and determine whether the heart rate has increased only because of the sensory stimulation. The correction term may be experimentally obtained and may be a time function. When the determination need not be very strict, the correction term may be omitted. $T_a$ is a certain threshold. The threshold may be experimentally obtained or may be zero.

When Expression (3) is true, the process proceeds to step 570A, and if not, the process proceeds to step 580A.

570A denotes a step of determining that the user has been aroused. This step may include a step of performing certain processing where, for example, the flavor inhaler or the like 200 issues a certain notification.

580A denotes a step of determining whether to end the determination whether the user has been aroused. Any reference may be used for the determination whether to end the determination whether the user has been aroused. It may be determined that the determination whether the user has been aroused is to end, for example, when it has been determined that the user's inhalation has ended, when heating of the flavor inhaler or the like 200 has ended, or when the certain operation has been performed on the input device such as a button or a switch. Alternatively, it may be determined that the determination whether the user has been aroused is to end, for example, when it has been determined that the user has taken a certain number of puffs since it was determined that the determination whether the user had been aroused was to start. Alternatively, it may be determined that the determination whether the user has been aroused is to end, for example, when a certain period of time has elapsed since it was determined the determination whether the user had been aroused was to start. If it is determined that the determination whether the user has been aroused is to end, the process ends, and if not, the process returns to step 530A.

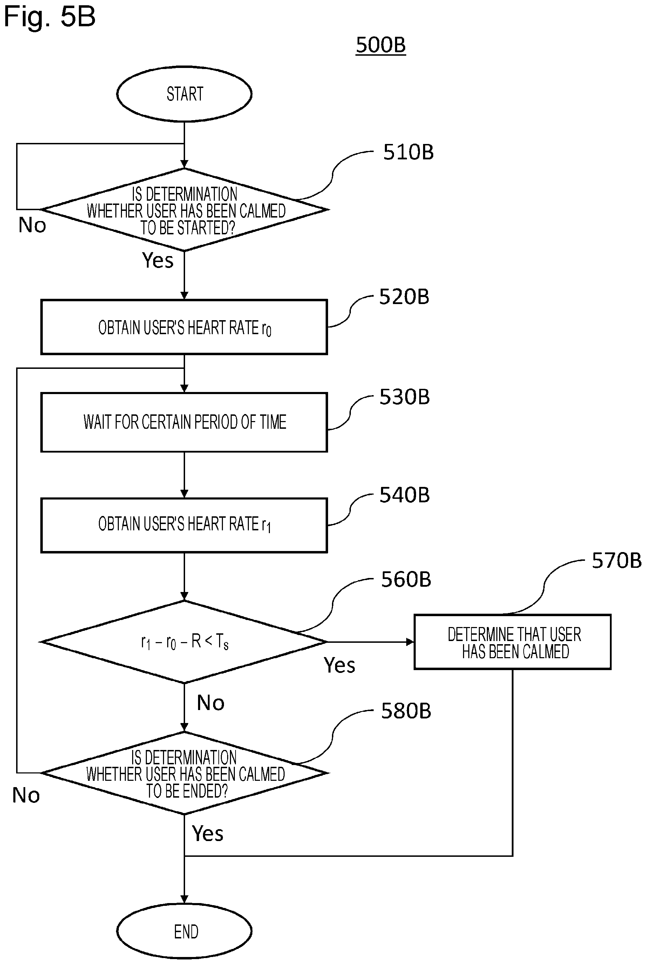

As a result of the sensory stimulation, the controller 240 can perform a process for determining whether the user has been calmed. Fig. 5B is a flowchart illustrating an exemplary process 500B performed by the controller 240 to determine whether the user has been calmed. The exemplary process 500B may be performed in parallel with the exemplary process 400.

510B denotes a step of determining whether to start a determination whether the user has been calmed. Any reference may be used for a determination whether to start the determination whether the user has been calmed. When the flavor inhaler or the like 200 has entered the flavor inhaler or the like 200, for example, it may be determined that the determination whether the user has been calmed is to start. Alternatively, it may be determined that the determination whether the user has been calmed is to start, for example, when the flavor inhaler or the like 200 is in the calming mode, when it has been determined that the user's inhalation has started, when heating of the flavor inhaler or the like 200 has started, or when the certain operation has been performed on the input device such as a button or a switch. If it is determined that the determination whether the user has been calmed is to start, the process proceeds to step 520B, and if not, the process returns to step 510B.

520B to 540B may be the same as steps 510A to 540A, respectively. A certain period of time in step 530B may be the same as or different from that in step 530A.

550B denotes a step of comparing the heart rate $r_0$ obtained in step 520B and the heart rate ri obtained in step 540B and determining whether the heart rate has decreased.

More specifically, comparison based on the following expression can be performed in this step.

$$r_1 - r_0 - R < T_s \qquad (4)$$

Here, a correction term R may be omitted when the determination need not be very strict. $T_s$ is a certain threshold. The threshold may be experimentally obtained or may be zero.

When Expression (4) is true, the process proceeds to step 570B, and if not, the process proceeds to step 580B.

570B denotes a step of determining that the user has been calmed. This step may include a step of performing certain processing where, for example, the flavor inhaler or the like 200 issues a certain notification.

580B denotes a step of determining whether to end the determination whether the user has been calmed. Any reference may be used for the determination whether the user has been calmed. It may be determined that the determination whether the user has been calmed is to end, for example, when it has been determined that the user's inhalation has ended, when heating of the flavor inhaler or the like 200 has ended, or when the certain operation has been performed on the input device such as a button or a switch. Alternatively, it may be determined that the determination whether the user has been calmed is to end, for example, when the user has taken a certain number of puffs since it was determined that the determination whether the user had been calmed was to start. Alternatively, it may be determined that the determination whether the user has been calmed is to end, for example, when a certain period of time has elapsed since it was determined that the determination whether the user had been calmed was to start. If it is determined that the determination whether the user has been calmed is to end, the process ends, and if not, the process returns to step 530B.

As is clear to those skilled in the art, whether the user has been aroused or calmed may be determined on the basis of the heart rate or fluctuations in heartbeat intervals.

The controller 240, therefore, may measure the user's heart rate a plurality of times using the heart rate sensor 220, obtain measurement data in a frequency domain by performing a Fourier transform on a plurality of heart rates measured (measurement data in a time domain), and perform a process for determining whether the user has been aroused or calmed on the basis of a frequency component included in the measurement data in the frequency domain.

The controller 240 may obtain measurement data in the frequency domain by performing a Fourier transform on a plurality of heart rates measured (measurement data in the time domain) obtained from an external device and perform a process for determining whether the user has been aroused or clamed on the basis of a frequency component included in the measurement data in the frequency domain. The external device or the like may generate the measurement data in the frequency domain, and the controller 240 may obtain the measurement data in the frequency domain from the external device.

2 Another Embodiment of Present Invention

Another embodiment of the present invention is a control method for the flavor inhaler or the like 200. The control method may include the above-described exemplary processes including the exemplary process 400.

Yet another embodiment of the present invention is a program causing a processor of the flavor inhaler or the like 200 to perform the control method.

3 Endnote

Although embodiments of the present invention have been described, the present invention is not limited to the above-described embodiments, and it is needless to say that the present invention may be implemented in various different modes without deviating from the scope of the technical idea thereof.

In addition, the scope of the present invention is not limited to the illustrated and described exemplary embodiments and encompasses all embodiments that produce effects equivalent to those aimed at by the present invention. Furthermore, the scope of the present invention is not limited to a combination of features of the invention defined by the claims and can be defined by every desired combination of certain ones of all the disclosed features.

REFERENCE SIGNS LIST

100A, 100B, 200, 300A, 300B flavor inhaler or the like
110 power supply unit
111A, 111B power supply
112A, 112B sensor
113A, 113B notifier
114A, 114B memory
115A, 115B communicator
116A, 116B, 240 controller
120 cartridge
121A, 121B heater
122 liquid guide
123 liquid storage
124,310B mouthpiece
130 flavor imparting cartridge
131 flavor source
140 holder
141 internal space
142 opening
143 bottom
144 heat insulator
150, 310A stick substrate
151 substrate
152 inhalation port
180 airflow path
181 air inlet hole
182 air outlet hole
210 sensory stimulation device
220 heart rate obtaining unit
315B button
320A, 320B range within which heart rate sensor is provided

**Claims**

1. A device that is a flavor inhaler or an aerosol generating device, the device comprising:

    a sensory stimulation device;
    a heart rate obtaining unit for obtaining a user's heart rate; and
    a controller for operating the sensory stimulation device,
    wherein the sensory stimulation device operates cyclically, and
    wherein the controller is configured to operate the sensory stimulation device such that a cycle of the operation of the sensory stimulation device is based on the heart rate obtained by the heart rate obtaining unit.

2. The device according to claim 1,
wherein the heart rate obtaining unit is a heart rate sensor.

3. The device according to claim 2,
wherein the heart rate sensor is a pulse wave sensor.

4. The device according to claim 1,
wherein the heart rate obtaining unit is a communicator configured to obtain the heart rate from an external device.

5. The device according to any of claims 1 to 4,
wherein the cycle of the operation of the sensory stimulation device is within a range of 80% to 120% of a cycle corresponding to the obtained heart rate.

6. The device according to any of claims 1 to 5,

   wherein an arousal mode and a calming mode are provided, and
   wherein the controller is also configured to operate the sensory stimulation device such that the cycle of the operation of the sensory stimulation device becomes, in the arousal mode, shorter than a cycle corresponding to the obtained heart rate and, in the calming mode, longer than the cycle corresponding to the obtained heart rate.

7. The device according to claim 6,
wherein operation intensity of the sensory stimulation device in the arousal mode is greater than operation intensity of the sensory stimulation device in the calming mode.

8. The device according to any of claims 1 to 7, configured to determine whether the user has been aroused and/or calmed by comparing the heart rate at a beginning of the obtaining and the heart rate at an end of the obtaining, and to notify of a result of the determination.

9. The device according to any of claims 1 to 8,
wherein a period of time for which the sensory stimulation device operates is longer than or equal to 0.05 second and shorter than or equal to 0.5 second in one cycle of the operation of the sensory stimulation device.

10. The device according to any of claims 1 to 9,
wherein the sensory stimulation device is a vibrator, a light-emitting device, and/or an acoustic device.

11. The device according to any of claims 1 to 10,
wherein the heart rate obtained by the heart rate obtaining unit is a moving average of the user's heart rate measured in a certain period of time.

12. A method for operating a device that is a flavor inhaler or an aerosol generating device, the device comprising:

   a sensory stimulation device,
   a heart rate obtaining unit for obtaining a user's heart rate, and
   a control unit for operating the sensory stimulation device,
   wherein the sensory stimulation device operates cyclically,
   the method comprising:
   a step of operating, by the controller, the sensory stimulation device such that a cycle of the operation of the sensory stimulation device is based on the heart rate obtained by the heart rate obtaining unit.

13. A program causing a processor of a flavor inhaler or an aerosol generating device to perform the method according to claim 12.

## Fig. 1A

100A

## Fig. 1B

POWER SUPPLY — 111B

SENSOR — 112B

NOTIFIER — 113B

MEMORY — 114B

COMMUNICATOR — 115B

CONTROLLER — 116B

144  100B

121B

151  152  150

142

143  140  141

EP 4 427 613 A1

# Fig. 2

200

| SENSORY STIMULATION DEVICE | 210 |
| HEART RATE OBTAINING UNIT | 220 |
| CONTROLLER | 240 |

Fig. 3A

# Fig. 3B

310B

300B

315B

320B

# Fig. 4

400

```
        ┌─────────────┐
        │    START    │
        └─────────────┘
               │
               ▼
     ◇ IS SENSORY              ─── 410
  No ◇ STIMULATION TO BE
     ◇ STARTED?
               │ Yes
               ▼
  ┌────────────────────────┐   ─── 420
  │ OBTAIN USER'S HEART RATE│
  └────────────────────────┘
               │
               ▼
  ┌────────────────────────┐   ─── 430
  │ SET CYCLE OF OPERATION ON│
  │ BASIS OF OBTAINED HEART RATE│
  └────────────────────────┘
               │
               ▼
  ┌────────────────────────┐   ─── 440
  │ OPERATE SENSORY STIMULATION│
  │ DEVICE WITH SET CYCLE  │
  └────────────────────────┘
               │
               ▼
     ◇ IS SENSORY              ─── 450
     ◇ STIMULATION TO BE   No
     ◇ ENDED?
               │ Yes
               ▼
        ┌─────────────┐
        │     END     │
        └─────────────┘
```

## Fig. 5A

500A

START

IS DETERMINATION WHETHER USER HAS BEEN AROUSED TO BE STARTED? — 510A

No

Yes

OBTAIN USER'S HEART RATE $r_0$ — 520A

WAIT FOR CERTAIN PERIOD OF TIME — 530A

OBTAIN USER'S HEART RATE $r_1$ — 540A

560A

570A

$r_1 - r_0 - R > T_a$

Yes → DETERMINE THAT USER HAS BEEN AROUSED

No

580A

IS DETERMINATION WHETHER USER HAS BEEN AROUSED TO BE ENDED?

No

Yes

END

## Fig. 5B

500B

```
        ( START )
            │
            ▼
   ┌──────────────────────┐ ──── 510B
No │ IS DETERMINATION     │
◄──│ WHETHER USER HAS BEEN│
   │ CALMED TO BE STARTED?│
   └──────────────────────┘
            │ Yes
            ▼
   ┌──────────────────────┐ ──── 520B
   │ OBTAIN USER'S HEART  │
   │ RATE r0              │
   └──────────────────────┘
            │
            ▼
   ┌──────────────────────┐ ──── 530B
   │ WAIT FOR CERTAIN     │
   │ PERIOD OF TIME       │
   └──────────────────────┘
            │
            ▼
   ┌──────────────────────┐ ──── 540B
   │ OBTAIN USER'S HEART  │
   │ RATE r1              │
   └──────────────────────┘
            │
            ▼
   ┌──────────────────────┐ ──── 560B          ──── 570B
   │  r1 – r0 – R < Ts    │  Yes   ┌─────────────────────┐
   │                      ├───────►│ DETERMINE THAT USER │
   └──────────────────────┘        │ HAS BEEN CALMED     │
            │ No                    └─────────────────────┘
            ▼
   ┌──────────────────────┐ ──── 580B
No │ IS DETERMINATION     │
◄──│ WHETHER USER HAS BEEN│
   │ CALMED TO BE ENDED?  │
   └──────────────────────┘
            │ Yes
            ▼
        ( END )
```

Heart rate calculations:

$$r_1 - r_0 - R < T_s$$

## INTERNATIONAL SEARCH REPORT

| | International application No. |
|---|---|
| | **PCT/JP2021/040355** |

| A. | CLASSIFICATION OF SUBJECT MATTER |
|---|---|

*A24F 40/53*(2020.01)i
FI:  A24F40/53

According to International Patent Classification (IPC) or to both national classification and IPC

| B. | FIELDS SEARCHED |
|---|---|

Minimum documentation searched (classification system followed by classification symbols)

A24F40/53

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2021-500002 A (RAI STRATEGIC HOLDINGS INC.) 07 January 2021 (2021-01-07) paragraph [0071] | 1-13 |
| A | JP 2020-68739 A (JAPAN TOBACCO INC.) 07 May 2020 (2020-05-07) paragraph [0052] | 1-13 |
| A | JP 2018-536388 A (NICOVENTURES HOLDINGS LTD.) 13 December 2018 (2018-12-13) fig. 4 | 1-13 |

☐ Further documents are listed in the continuation of Box C.       ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 December 2021** | **14 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2021/040355**

| Patent document cited in search report | | | Publication date (day/month/year) | Patent family member(s) | | | Publication date (day/month/year) |
|---|---|---|---|---|---|---|---|
| JP | 2021-500002 | A | 07 January 2021 | US | 2018/0338527 | A1 | |
| | | | | paragraph [0072] | | | |
| | | | | WO | 2018/215939 | A1 | |
| | | | | KR | 10-2020-0011961 | A | |
| | | | | CN | 111372511 | A | |
| JP | 2020-68739 | A | 07 May 2020 | KR | 10-2020-0050868 | A | |
| | | | | paragraphs [0118]-[0120] | | | |
| | | | | CN | 111134365 | A | |
| JP | 2018-536388 | A | 13 December 2018 | US | 2019/0053540 | A1 | |
| | | | | fig. 4 | | | |
| | | | | WO | 2017/055795 | A1 | |
| | | | | KR | 10-2018-0044410 | A | |
| | | | | CN | 108135267 | A | |

Form PCT/ISA/210 (patent family annex) (January 2015)

21

**EP 4 427 613 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021500002 W